# FASCICULE DE BREVET EUROPEEN

(11) **EP 1 007 522 B1**
(45) Date de publication et mention de la délivrance du brevet: **20.11.2002**
(21) Numéro de dépôt: 98967138.3
(22) Date de dépôt: 28.05.1998
(51) Int. Cl.: C07D 403/12, A61K 31/415, A61K 31/505

(54) **2- 4-[4-(4,5-DICHLORO-2-METHYLIMIDAZOL-1-YL)BUTYL]PIPERAZIN-1-YL -5-FLUOROPYRIMIDINE, SA PREPARATION ET SON UTILISATION THERAPEUTIQUE**
2- 4-[4-(4,5-DICHLOR-2-METHYLIMIDAZOL-1-YL)BUTYL]PIPERAZIN-1-YL -5-FLUORPYRIMIDIN, SEINE HERSTELLUNG UND THERAPEUTISCHE VERWENDUNG
2- 4-[4-(4,5-DICHLORO-2-METHYLIMIDAZOL-1-YL)BUTYL]PIPERAZIN-1-YL -5-FLUOROPYRIMIDINE, ITS PREPARATION AND THERAPEUTIC USE

(30) Priorité: 02.06.1997 FR 9706738
(43) Date de publication de la demande: 14.06.2000
(73) Titulaire: LABORATORIOS DEL DR. ESTEVE, S.A., 08026 Barcelona (ES)
(72) Inventeur: MERCE-VIDAL, Ramon, E-08014, Barcelone (ES); FRIGOLA-CONSTANSA, Jordi, E-08013, Barcelone (ES)
(74) Mandataire: Ahner, Francis
(86) Numéro de dépôt international: EP9803190
(87) Numéro de publication internationale: WO98055476

(56) Documents cités:
- EP-A- 0 382 637
- EP-A- 0 497 659
- EP-A- 0 655 248
- WO-A-97/21439

## Description

La présente invention concerne le nouveau composé 2-{4-[4-(4,5-dichloro-2-méthylimidazol-1-yl)butyl]-1-pipérazinyl}-5-fluoropyrimidine, sa préparation et son utilisation en thérapeutique, ainsi que ses sels physiologiquement acceptables, les procédés pour leur préparation, et leur application comme médicament en thérapeutique humaine et/ou vétérinaire, et les compositions pharmaceutiques qui les contiennent.

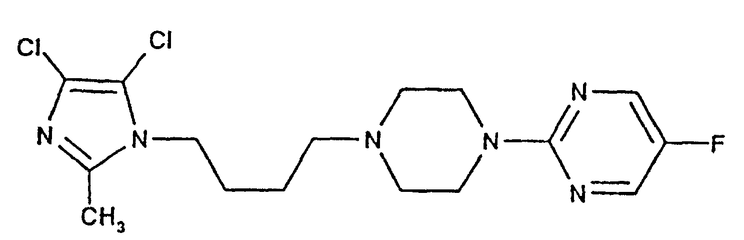

Dans les brevets EP 382 637 et EP 497 659 de la demanderesse, on a décrit divers dérivés de pyrimidinyl-pipérazinyl-alkyl-azoles ayant des propriétés anxiolytiques et/ou tranquillisantes. Bien que le brevet EP 382 637 revendique les dérivés pyrimidinyle-pipérazinyl-alkyl-azoles substitués en position 5 de la pyrimidine par un atome d'halogène, seuls deux exemples de ce type de composés sont décrits, et dans les deux cas il s'agit d'un atome de brome.

La demanderesse a maintenant découvert que l'introduction d'un atome de fluor comme substituant en position 5 de la pyrimidine et dans le cas spécial où l'azole est un imidazole trisubstitué par un groupe méthyle en position 2 et par deux atomes de chlore en positions 4 et 5, donne naissance au composé objet du présent brevet qui présente quelques propriétés biologiques intéressantes le rendant particulièrement utile pour son application en thérapeutique humaine et/ou vétérinaire. En particulier, le composé objet du présent brevet est utile comme antiémétique, contre le mal de mer (nausées provoquées par le mouvement), comme anti-dépressif, anxiolytique, comme inhibiteur de la sécrétion d'acide gastrique, les maladies obsessivo-compulsives, dans les attaques de panique et en apnée du sommeil, chez les mammifères, y compris l'homme.

On peut préparer le composé 2-{4-[4-(4,5-dichloro-2-méthylimidazol-1-yl)butyl]-1-pipérazinyl}-5-fluoropyrimidine ainsi que ses sels physiologiquement acceptables, selon l'invention, par l'un des procédés indiqués ci-dessous.
a) par réaction de 8-(5-fluoro-2-pyrimidinyl)-8-aza-5-azoniaspiro[4,5]décane avec le 4,5-dichloro-2-méthyl-1H-imidazole en présence de carbonate de potassium et dans un solvant dipolaire aprotique comme le diméthylformamide. La température de réaction peut varier entre 70°C et la température de reflux du solvant, et le temps de réaction oscille entre 3 et 48 heures. 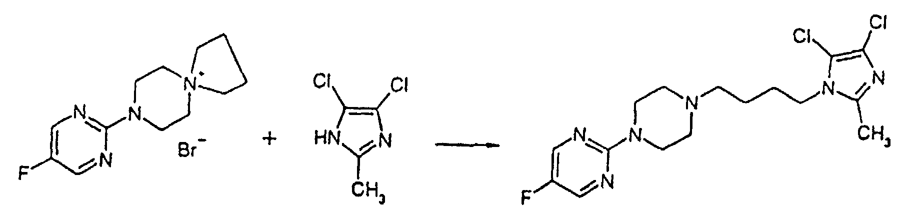
b) Par réaction de la 5-fluoro-2-(pipérazin-1-yl)-pyrimidine avec le 1-(4-chlorobutyl)-4,5-dichloro-2-méthyl-1H-imidazole en présence de carbonate de potassium et dans un solvant dipolaire aprotique comme le diméthylformamide. La température de réaction peut varier entre 70°C et la température de reflux du solvant, et le temps de réaction varie entre 3 et 48 heures. On peut également procéder à cette réaction dans des conditions de transfert de phase en utilisant une solution aqueuse d'hydroxyde de sodium, du toluène et un catalyseur comme le bromure de tétrabutylammonium. Dans ces conditions la température de la réaction peut varier entre 50°C et 90°C, et le temps de réaction varie entre 12 et 72 heures. 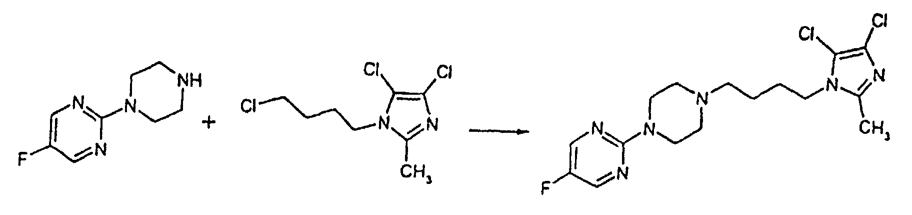
c) Par la réaction de 2-{4-[4-(4,5-dichloro-2-méthylimidazol-1-yl)butyl]pipérazin-1-yl}-5-fluoropy-rimidine et d'un acide physiologiquement acceptable comme l'acide citrique dans un solvant adéquat comme l'éthanol.

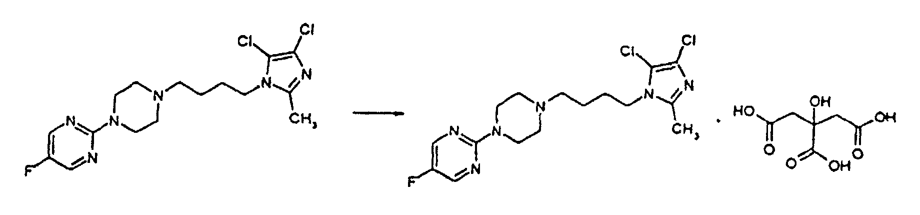

Dans les exemples suivants, on indique la préparation du nouveau composé objet de l'invention ainsi que de ses sels physiologiquement acceptables. On décrit également quelques activités biologiques et quelques formes d'emploi. Les exemples indiqués ci-dessous, donnés à simple titre d'illustration, ne doivent cependant en aucune manière limiter la portée de l'invention.

### Exemple 1 - Préparation de 2-{4-[4-(4,5-dichloro-2-méthylimidazol-1-yl)butyl]pipérazin-1-yl}-5-fluoropyrimidine

On maintient au reflux pendant 12 heures un mélange de 3,17 g (0,01 mole) de 8-(5-fluoro-2-pyrimidinyl)-8-aza-5-azoniaspiro[4,5]décane, 2,26 g (0,015 mole) de 4,5-dichloro-2-méthyl-1H-imidazole et de 76 g (0,02 mole) de carbonate de potassium dans 80 ml de diméthylformamide. Ensuite, on fait évaporer à siccité et on redissout le produit brut résultant dans le chloroforme et on le lave de façon répétée avec de l'eau. On sèche la phase organique et on la fait évaporer, puis on purifie le produit brut résultant par chromatographie sur colonne de gel de silice. On obtient 3,3 g (rendement 85%) de 2-{4-[4-(4,5-dichloro-2-méthylimidazol-1-yl)butyl]pipérazin-1-yl}-5-fluoropyrimidine sous forme d'huile.
IR (film), cm⁻¹ : 2944, 1610, 1555, 1503, 1449, 1402, 1361, 1243, 786.
RMN'-H (CDCl₃, 300 MHz), δ : 1,54 (m,2H), 1,73 (m,2H), 2,34 (s,3H), 2,38 (m,2H), 2,43 (m,4H), 3,74 (m,4H), 3,85 (m,2H), 8,15 (s,2H).

### Exemple 2 - Préparation de 2-{4-[4-(4,5-dichloro-2-méthylimidazol-1-yl)butyl]pipérazin-1-yl}-5-fluoropyrimidine

On maintient au reflux pendant 12 heures un mélange de 3,5 g (0,02 mole) de 5-fluoro-2-(pipérazin-1-yl)-pyrimidine, 6,04 g (0,025 mole) de 1-(4-chlorobutyl)-4,5-dichloro-2-méthyl-1H-imidazole et de 4,14 g (0,03 mole) de carbonate de potassium dans 200 ml de diméthylformamide. Ensuite on fait évaporer à siccité et on redissout le produit brut résultant dans le chloroforme et on le lave de façon répétée avec de l'eau. On sèche la phase organique et on la fait évaporer, puis on purifie le produit brut résultant par chromatographie sur colonne de gel de silice. On obtient 6,4 g (rendement 83%) de 2-{4-[4-(4,5-dichloro-2-méthylimidazol-1-yl)butyl]pipérazin-1-yl}-5-fluoropyrimidine sous forme d'huile.
IR (film), cm⁻¹ : 2944, 1610, 1555, 1503, 1449, 1402, 1361, 1243, 786.
RMN'-H (CDCl₃, 300 MHz), δ : 1,54 (m,2H), 1,73 (m,2H), 2,34 (s,3H), 2,38 (m,2H), 2,43 (m,4H), 3,74 (m,4H), 3,85 (m,2H), 8,15 (s,2H).

### Exemple 3 - Préparation du citrate de 2-{4-[4-(4,5-dichloro-2-méthylimidazol-1-yl)butyl]pipérazin-1-yl}-5-fluoropyrimidine

A une solution de 2 g (5,2 mole) de 2-{4-[4-(4,5-dichloro-2-méthylimidazol-1-yl)butyl]pipérazin-1-yl}-5-fluoropyrimidine dans l'éthanol absolu on ajoute 1,1 g (5,2 mmoles) d'acide citrique monohydraté. Au bout d'un certain temps, 2,72 g (rendement 91%) de citrate de 2-{4-[4-(4,5-dichloro-2-méthylimidazol-1-yl)butyl]pipérazin-l-yl}-5-fluoropyrimidine précipitent sous forme de solide de P.f. 151-153°C.
IR(KBR), cm⁻¹ : 3780-2270 (bb), 1707, 1616, 1482, 1429, 1375, 1244, 1214.
RMN'-H (DMSO-d₆, 300 MHz), δ : 1,53 (m,2H), 1,66 (m,2H), 2,33 (s,3H), 2,53-2,74 (a.c., 10H), 3,75 (m,4H), 3,94 (t,J=7,2 Hz,2H), 8,46 (s,2H).

### ACTIVITE ANTIEMETIQUE :

On étudie les composés obtenus dans l'invention quant à leurs effets sur la nausée sur le furet selon un procédé décrit par Costall et al (*Neuropharmacology*, **1986**, 25,959-961).

On loge individuellement des furets des deux sexes (0,7 - 1,4 kg) à 21 ± 1°C et on les alimente normalement. Ensuite on leur administre le composé de l'exemple ou un véhicule par voie sous-cutanée comme prétraitement de 15 minutes avant l'administration de sulfate de cuivre (CuSO₄.5H₂O ; 100 mg/kg par voie intragastrique) ou de cisplatine (10 mg/kg i.v. au moyen d'une canule jugulaire fixe). On observe les animaux au commencement du vomissement, puis pendant 30 minutes (sulfate de cuivre) ou 240 minutes (cisplatine). Le vomissement se caractérise par des contractions abdominales rythmiques, soit associées à l'expulsion de matières solides ou liquides (vomissements), soit non associées au passage de matières par la bouche (nausées). On enregistre le nombre d'épisodes et les nausées ou vomissements.

Le composé de l'exemple 3 est capable de modifier la nausée induite par le sulfate de cuivre et de s'opposer à la nausée induite par la cisplatine.
a) Sulfate de cuivre

| Composé | Dose(mg/kg s.c.) | Nombre de nausées/ vomissements |
|---|---|---|
| Véhicule | - | 8,2 ± 1,4 |
| Exemple 3 | 0,001 | 7,4±1,2(NS) |
| | 0,01 | 3,2 ± 0,6 (*) |
| | 0,1 | 1,0 ± 0,2 (*) |
| | 1 | 1.7 ± 0,3 (*) |

| | | |
|---|---|---|
| (*) p < 0,05 comparé avec les valeurs du véhicule témoin | | |

b) Cisplatine

| Composé | Dose(mg/kg s.c.) | Nombre de nausées/ vomissements |
|---|---|---|
| Véhicule | - | 14,2 ± 1,9 |
| Exemples 3 | 0,001 | 13,8 ± 2,4 (NS) |
| | 0,01 | 6,5±3,1(*) |
| | 0,1 | 3,1±2,9(*) |
| | 1 | 3,0±2,0(*) |

| | | |
|---|---|---|
| (*) p < 0,05 comparé avec les valeurs du véhicule témoin | | |

### ETUDE DU MAL DES TRANSPORTS CHEZ LA MUSARAIGNE (SUNCUS MURINUS)

On étudie le composé de l'exemple 3 quant à son effet sur le mal des transports (vertige) provoqué chez *Suncus murinus* selon le procédé décrit par N.Matsuki et al. ("Mechanisms and control of emesis", John Libbey Emotex, **1992**, 233, 323-329).

On loge les *Suncus murinus*, mâles, pesant 58-72 grammes, par groupes de 3 et on leur laisse libre accès à un aliment solide et à de l'eau. Une heure avant l'épreuve on leur présente de la nourriture pour chats dans leur cage de logement. Au début de l'épreuve les animaux recoivent le traitement avec le produit à essayer par voie i.p. Au bout de 30 minutes on les met individuellement dans les boîtes d'essai (15x15x10 cm). Les boîtes sont reliées à un moteur qui les déplace sur plusieurs roues vers l'avant et vers l'arrière, avec une fréquence qui leur cause une nausée (mal des transports). Cette fréquence est de 1 Herz avec une amplitude de déplacement de 4 cm. On laisse les animaux 30 minutes dans les boîtes d'essai pour qu'ils s'habituent, puis on commence le mouvement actif des cages pendant 20 minutes, pour déterminer la présence ou l'absence de nausées (sans passage de matière de l'appareil digestif) et de vomissements (avec passage de matière de l'appareil digestif).

Le composé de l'exemple 3 inhibe nettement la nausée provoquée par le mouvement (mal des transports) réduisant aussi bien les nausées que les vomissements, entre des doses de 0,01 et 10 mg/kg, par voie i.p.

| -Composé | Dose(mg/kg i.p.) | Nombre de nausées/ vomissements |
|---|---|---|
| Véhicule | - | 10,0 ± 1,39 |
| Exemple 3 | 0,001 | 8,38 ± 1,41 (NS) |
| | 0,01 | 0,33 ± 0,21 (*) |
| | 0,1 | 2,17 ± 0,83 (*) |
| | 1,0 | 0,33 ± 0,21 (*) |
| | 10,0 | 0 ± 0 (*) |

| | | |
|---|---|---|
| (*) p < 0,0001 comparé avec les valeurs du véhicule témoin | | |

### ACTIVITE ANXIOLYTIQUE ET ANTIDEPRESSIVE

On démontre l'activité anxiolytique et antidépressive par l'affinité que présentent les nouveaux composés pour les récepteurs de la sérotonine 5HT_{1A} (D.A. GLitz, *Drugs,* **1991**, 41, 11) et au moyen de l'épreuve de réponse d'évitement conditionné (J.S. New et al., J.Med.Chem., **1986,** 29, 1476).

### a) Liaison au récepteur de sérotonine 5HT_{1A}

On utilise un homogénéisat d'hippocampe de rat en suivant une modification du procédé de S.J.Peroutka *(Neurochem,* **1986,** 47, 529). Comme radiocoordinat on utilise [³H]-8-OH-DPAT, et pour mesurer l'union non spécifique on utilise de la sérotonine. Le temps d'incubation est de 15 minutes à une température de 37°C. On sépare le radiocoordinat uni à la protéine par flitration sur filtres de fibre de verre et on détermine la radioactivité retenue sur le filtre par scintillation liquide. On calcule la constante d'inhibition (Kᵢ, nM) par analyse de régression non linéaire en utilisant le programme EBDA/LIGAND (Munson et Rodbard, *Analytical Biochemistry*, **1980,** 107, 220).
Composé de l'exemple 3 Kᵢ = 19,4 nM

### b) Test de la réponse d'évitement conditionnée (C.A.R.)

Dans cette épreuve, on utilise des rats Wistar, mâles, pesant 200 g, entraînés pour sauter une barrière dans une cage Letica (références LI 910 et LI 2700) d'évitement et de sortie (shuttle box) pendant les 30 secondes qui suivent leur introduction dans la cage.

Les produits ayant une activité anxiolytique ou tranquillisante suppriment la réponse d'évitement conditionnée.

Entraînement : premier jour, 11 essais à intervalles de 3 minutes. Electrochoc dans les pattes à 30 secondes (5 mA, 0,1 s, 10 s). Second et troisième jours : deux essais quotidiens, uniquement avec les rats sélectionnés {certaines des notes du premier jour (sauf le premier essai) > 14}. Jour de l'essai : groupes formés par les rats sélectionnés. Administration orale du produit ou du véhicule 45 minutes avant le commencement de l'étude.
Composé de l'exemple 3 DE₅₀ = 9,7 mg/kg, p.o.

### ACTIVITE INHIBITRICE DE LA SECRETION D'ACIDE GASTRIQUE

On détermine cette activité au moyen du procédé de Shay (H.Shay, et al., *Gastroenterology*, **1945,** 5, 43 ; Visscher et al., *J. Pharmac. Exp. Ther*., **1954**, 110, 188).

Dans cette épreuve on utilise des rats Wistar, mâles, pesant de 200 à 250 g, que l'on maintient a jeun jusqu'au jour précédant l'épreuve, avec accès libre à l'eau. On utilise, au minimum, des lots de 4 animaux chacun.

On anesthésie les rats avec de l'éther éthylique, on leur pratique une laparotomie et on leur lie le pylore, puis on effectue une incision abdominale. On procède à l'administration des produits par voie intraduodénale (i.d.) avant de coudre l'incision abdominale. La dose administrée dans le premier essai est de 40 mg/kg, puis on détermine la dose efficace médiane (DE₅₀). Le véhicule utilisé est de la gomme arabique à 5% p/v dans de l'eau bidistillée.

Deux heures après la ligature du pylore, on sacrifie les rats par anesthésie prolongée avec de l'éther éthylique et on mesure le volume de suc gastrique, on détermine l'acidité totale au moyen d'un pH-mètre muni d'une burette automatique. Pour chaque produit et pour chaque dose essayée on détermine le pourcentage d'inhibition de la sécrétion d'acide gastrique par rapport à un lot de référence.
Composé de l'exemple 3 DE₅₀ = 1,9 mg/kg,i.d.

La posologie quotidienne en médecine humaine ou vétérinaire est comprise entre 1 mg et 500 mg de produit que l'on peut administrer en une ou plusieurs prises. On prépare les compositions sous des formes compatibles avec la voie d'administration utilisée, comme par exemple des comprimés, dragées, gélules, suppositoires, solutions ou suspensions. On prépare ces compositions au moyen de procédés connus, et elles comprennent de 1 à 60% en poids du principe actif et de 40 à 99% en poids de véhicule pharmaceutique approprié et compatible avec le principe actif et la forme physique de la composition appropriée. A titre d'exemple, on présente la formule de trois formes galéniques qui contiennent un produit de l'invention.

| Exemple de formulation pour comprimé | |
|---|---|
| Exemple 3 | 5 mg |
| Lactose | 60 mg |
| Cellulose cristalline | 25 mg |
| Povidone K 90 | 5 mg |
| Amidon prégélatinisé | 3 mg |
| Dioxyde de silice colloïdal | 1 mg |
| Stéarate de magnésium | 1 mg |
| Poids total | 100 mg |

| Exemple de formulation pour gélule | |
|---|---|
| Exemple 3 | 10 mg |
| Glycéride polyoxyéthylénique | 135 mg |
| Béhénate de glycérine | 5 mg |
| Excipient | 150 mg |

| Exemple de formulation pour préparation injectable | |
|---|---|
| Exemple 3 | 4 mg 8 mg |
| Chlorure de sodium | 15 mg 30 mg |
| Eau injectable q.s.p. | 2 ml 4 ml |

## Revendications

1. Le composé 2-{4-[4-(4,5-dichloro-2-méthylimidazol-1-yl)butyl]-1-pipérazinyl}-5-fluoropyrimidine, ainsi que ses sels physiologiquement acceptables, de formule :

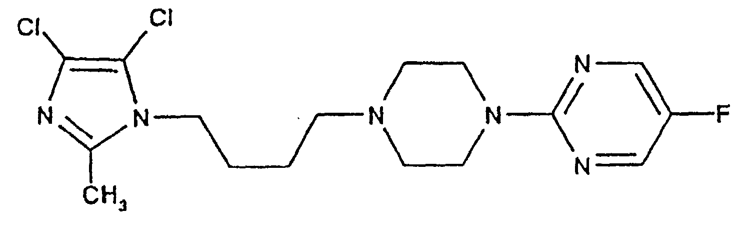

2. Le citrate de 2-{4-[4-(4,5-dichloro-2-méthylimidazol-1-yl)butyl]pipérazin-1-yl}-5-fluoropyrimidine.

3. Procédé de préparation des composés selon l'une des revendications 1 et 2, qui se **caractérise en ce qu'**on effectue l'une des opérations suivantes :
a) par réaction de 8-(5-fluoro-2-pyrimidinyl)-8-aza-5-azoniaspiro[4,5]décane avec le 4,5-dichloro-2-méthyl-1H-imidazole en présence de carbonate de potassium et dans un solvant dipolaire aprotique comme le diméthylformamide, la température de réaction pouvant varier entre 70°C et la température de reflux du solvant, et le temps de réaction pouvant varier entre 3 et 48 heures.
b) par réaction de 5-fluoro-2-(pipérazin-1-yl)-pyrimidine avec le 1-(4-chlorobutyl)-4,5-dichloro-2-méthyl-1H-imidazole en présence de carbonate de potassium et dans un solvant dipolaire aprotique comme le diméthylformamide, la température de réaction pouvant varier entre 70°C et la température de reflux du du solvant, et le temps de réaction pouvant varier entre 3 et 48 heures. On peut également procéder à cette réaction dans des conditions de transfert de phase en utilisant une solution aqueuse d'hydroxyde de sodium, du toluène et un catalyseur comme le bromure tétrabutylammonium. Dans ces conditions, la température de réaction peut varier entre 50°C et 90°C, et le temps de réaction varie entre 12 et 72 heures.
c) par réaction de 2-{4-[4-(4,5-dichloro-2-méthylimidazol-1-yl)butyl]pipérazin-1-yl}-5-fluoropyrimidine et d'un acide physiologiquement acceptable comme l'acide citrique dans un solvant approprié comme l'éthanol.

4. Utilisation des composés selon les revendications 1, 2 et 3, pour la préparation de médicaments destinés au traitement des vertiges, du mal des transports (nausées), de l'émèse, de la dépression, de l'anxiété, de la sécrétion d'acide gastrique, des désordres obsessifs ou compulsifs, des attaques de panique et de l'apnée du sommeil.

5. Compositions pharmaceutiques **caractérisées par le fait qu'**elles contiennent, outre un support pharmaceutiquement acceptable, le composé 2-{4-[4-(4,5-dichloro-2-méthylimidazol-1-yl)butyl]-1-pipérazinyl}-5-fluoropyrimidine, ou un de ses sels physiologiquement acceptables, selon l'une des revendications 1 et 2.

## Patentansprüche

1. Die Verbindung 2-{4-[4-(4,5-Dichlor-2-methylimidazol-1-yl)butyl]-1-piperazinyl}-5-fluorpyrimidin sowie ihre physiologisch annehmbaren Salze der Formel:

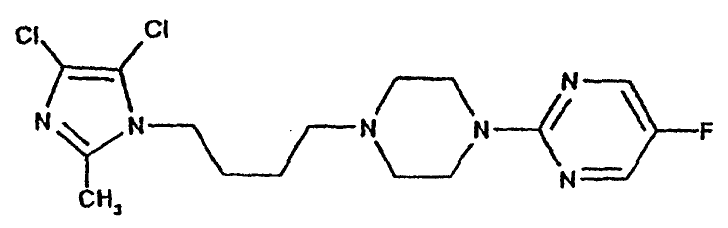

2. Citrat von 2-{4-[4-(4,5-Dichlor-2-methylimidazol-1-yl)butyl]piperazin-1-yl}-5-fluorpyrimidin.

3. Verfahren zur Herstellung der Verbindungen nach einem der Ansprüche 1 und 2, das **dadurch gekennzeichnet ist, dass** man eine der folgenden Vorgehensweisen bewirkt:
a) Umsetzung von 8-(5-Fluor-2-pyrimidinyl)-8-aza-5-azoniaspiro[4,5]decan mit 4,5-Dichlor-2-methyl-1H-imidazol in Anwesenheit von Kaliumcarbonat und in einem dipolaren aprotischen Lösungsmittel wie Dimethylformamid, wobei die Reaktionstemperatur zwischen 70 °C und der Rückflusstemperatur des Lösungsmittels variieren kann und die Reaktionsdauer zwischen 3 und 48 Stunden variieren kann.
b) Umsetzung von 5-Fluor-2-(piperazin-1-yl)pyrimidin mit 1-(4-Chlorbutyl)-4,5-dichlor-2-methyl-1H-imidazol in Anwesenheit von Kaliumcarbonat und in einem dipolaren aprotischen Lösungsmittel, wie Dimethylformamid, wobei die Reaktionstemperatur zwischen 70 °C und der Rückflusstemperatur des Lösungsmittels variieren kann und die Reaktionszeit zwischen 3 und 48 Stunden variieren kann. Man kann diese Umsetzung auch unter Phasentransfer-Bedingungen durchführen, indem man eine wässrige Lösung von Natriumhydroxid, Toluol und einen Katalysator, wie Tetrabutylammoniumbromid, verwendet. Unter diesen Bedingungen kann die Reaktionstemperatur zwischen 50 °C und 90 °C variieren, und die Reaktionszeit variiert zwischen 12 und 72 Stunden.
c) Umsetzung von 2-{4-[4-(4,5-Dichlor-2-methylimidazol-1-yl)butyl]piperazin-1-yl}-5-fluorpyrimidin und von einer physiologisch annehmbaren Säure, wie Zitronensäure, in einem geeigneten Lösungsmittel, wie Ethanol.

4. Verwendung von Verbindungen nach einem der Ansprüche 1 und 2 für die Herstellung von Medikamenten, die zur Behandlung von Schwindelanfällen, Reiseübelkeit (Nausea), Enbrechen, Depression, Angst, Magensäure-Sekretion, obsessiven oder Zwangsstörungen, Panikattacken und Schlafapnoe bestimmt sind.

5. Pharmazeutische Zusammensetzungen, **dadurch gekennzeichnet, dass** sie außer einem pharmazeutisch annehmbaren Träger die Verbindung 2-{4-[4-(4,5-Dichlor-2-methylimidazol-1-yl)butyl]-1-piperazinyl}-5-fluorpyrimidin oder eines ihrer physiologisch annehmbaren Salze gemäß einem der Ansprüche 1 und 2 enthalten.

## Claims

1. The compound 2-{4-[4-(4,5-dichloro-2-methylimidazol-1-yl)butyl]-1-piperazinyl}-5-fluoropyrimidine, and its physiologically acceptable salts, of formula:

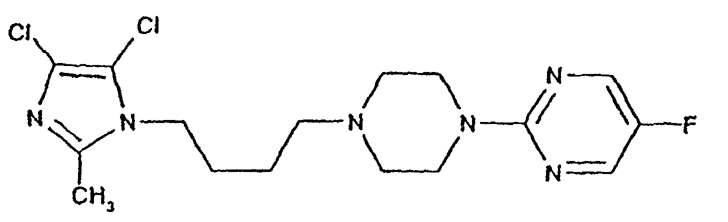

2. 2-{4-[4-(4,5-Dichloro-2-methylimidazol-1-yl)butyl]piperazin-1-yl}-5-fluoropyrimidine citrate.

3. Process for the preparation of the compounds according to either of Claims 1 and 2, which process is **characterized in that** one of the following operations is carried out:
a) reaction of 8-(5-fluoro-2-pyrimidinyl)-8-aza-5-azoniaspiro[4.5]decane with 4,5-diehloro-2-methyl-1H-imidazole in the presence of potassium carbonate and in a dipolar aprotic solvent, such as dimethylformamide, it being possible for the reaction temperature to vary between 70°C and the reflux temperature of the solvent and it being possible for the reaction time to vary between 3 and 48 hours,
b) reaction of 5-fluoro-2-(piperazin-1-yl)-pyrimidine with 1-(4-chlorobutyl)-4,5-dichloro-2-methyl-1H-imidazole in the presence of potassium carbonate and in a dipolar aprotic solvent, such as dimethylformamide, it being possible for the reaction temperature to vary between 70°C and the reflux temperature of the solvent and it being possible for the reaction time to vary between 3 and 48 hours. This reaction can also be carried out under phase transfer conditions by using an aqueous sodium hydroxide solution, toluene and a catalyst, such as tetrabutylammonium bromide; under these conditions, the temperature of the reaction can vary between 50°C and 90°C, and the reaction time varies between 12 and 72 hours,
c) reaction of 2-{4-[4-(4,5-dichloro-2-methylimidazol-1-yl)butyl]piperazin-1-yl}-5-fluoropyrimidine and of a physiologically acceptable acid, such as citric acid, in an appropriate solvent, such as ethanol.

4. Use of the compounds according to either of Claims 1 and 2 in the preparation of medicaments intended for the treatment of vertigo, travel sickness (nausea), emesis, depression, anxiety, gastric acid secretion, obsessive or compulsive disorders, panic attacks and sleep apnea.

5. Pharmaceutical compositions, **characterized in that** they comprise, in addition to a pharmaceutically acceptable vehicle, the compound 2-{4-[4-(4,5-dichloro-2-methylimidazol-1-yl)butyl]-1-piperazinyl}-5-fluoropyrimidine, or one of its physiologically acceptable salts, according to either of Claims 1 and 2.
